# EUROPEAN PATENT APPLICATION

(11) **EP 4 174 184 A1**
(43) Date of publication of application: **03.05.2023**
(21) Application number: 22203201.3
(22) Date of filing: 24.10.2022
(51) Int. Cl.: C12P 7/22, C12N 1/20

(54) **PSEUDOMONAS STRAIN FOR THE MANUFACTURE OF 1,5-DIHYDROXYNAPHTHALENE**

(30) Priority: 27.10.2021 WO PCT/CN2021/126620
(71) Applicant: Covestro Deutschland AG, 51373 Leverkusen (DE)
(72) Inventor: Guo, Ruijing, Shanghai, 201206 (CN); Lin, Jen-Chieh, 201114 Singapore (SG); Kequan, Chen, Nanjing, 211816 (CN); He, Xun, Nanjing, 211816 (CN); Li, Kang, Nanjing, 211816 (CN); Gao, Yang, Nanjing, 211816 (CN)
(74) Representative: Levpat

(57) **Abstract**

The present invention relates to a novel *Pseudomonas* strain for the manufacture of 1,5-dihydroxynaphthalene and methods for its use.

## Description

The present invention relates to a novel *Pseudomonas* strain for the manufacture of 1,5-dihydroxynaphthalene and methods for its use.

1,5-dihydroxynapthalene is a precursor for the manufacture of various chemical compounds, amongst them 1,5-naphthalene diisocyanate. It is currently produced by chemical synthesis based on petrochemical products, especially naphthalene and naphthalene-1-monosulfonic acid. The synthesis route from these compounds to 1,5-dihydroxynaphthalene is hampered by low yields, low purity of the product and the generation of many undesired by-products. Thus, there is the need for a process which for the production of 1,5-dihydroxynaphthalene with high yields and little energy consumption.

The manufacture of dihydroxynapthalene isomers, amongst them 1,5-dihydroxynaphthalene, by microbial oxidation of precursor molecules has been described by Bianchi et al., 1997, "Synthesis of Dihydroxynaphthalene Isomers by Microbial Oxidation of 1- and 2-Naphthol", Applied Microbiology and Biotechnology, 48: 363-366. The process employs *Pseudomonas putida.* Unfortunately, this organism has some disadvantages. *P. putida* only produces 1,5-dihydroxynaphthalene if provided with acetylsalicylic acid as inducer and glucose as additional substrate.

In the study underlying the present invention a strain of *Pseudomonas guariconensis njensis* was isolated which was able to convert 1-naphthol to 1,5-dihydroxynaphthalene without induction and without a cosubstrate. The capabilities of this strain have been improved by mutagenesis.

Therefore, in a first embodiment the present invention relates to a method for producing 1,5-dihydronaphthalene comprising the steps of
a) Adding microbial cells belonging to *Pseudomonas guariconensis njensis* to a fermentation broth comprising 1-Naphthol or Naphthalene or a mixture of 1-naphthol and napthalene; and
b) incubating the cells at a temperature between 30 and 40 °C for at least 3 hours.

The term *"Pseudomonas guariconensis njensis"* refers to the strain deposited as (deposited at the China Center for Type Culture Collection as CCTCC M 2021615). Based on the analysis of the sequence of the 16s rDNA the closest described relative of this strain is *Pseudomonas guariconensis* LMG 27394T (16s rDNA sequence deposited at Genbank as HF674459).

In a preferred embodiment of the present invention, *Pseudomonas guariconensis njensis* strain 2-40 deposited as (deposited at the China Center for Type Culture Collection as CCTCC M 2021614) is used in the method. This strain will also be referred to as *"P. njensis* 2-40". This strain has been derived from the wild type of *Pseudomonas guariconensis njensis* by random mutagenesis and shows increased titers of 1,5-dihydroxynaphthalene when incubated with 1-naphthol.

The fermentation broth can have any composition which allows growth of *Pseudomonas guariconensis njensis.* Preferred are those compositions which have been shown to support growth of *Pseudomonas sp.* in general. The suitability of a particular fermentation broth can easily be determined by simple growth experiments.

The carbon source is 1-naphthol and/or naphthalene with 1-naphtol being preferred.

As higher concentrations of naphthol are toxic and lead to decreased metabolic activity, it is preferred that the concentration of naphthol in the fermentation broth does not exceed 0.5 g/l, more preferably 0.2 g/l. The minimum concentration which is preferred to enable production of 1,5-dihydroxynaphthalene is 0.1 g/l. In order to obtain good yields of 1,5-dihydroxynapthalene despite the toxicity of the substrate the incubation step b) is preferably a fed-batch process, wherein 1-naphtol is added at least twice at different points in time so that each addition results in a concentration of 1-napthol in the fermentation broth which is between 0.1 and 0.5 g/l, preferably 0.2 and 0.3 g/l.

If 1-naphthol is used as carbon source, it is preferred that the fermentation broth additionally comprises at least one co-substrate selected from the group consisting of naphthalene, glycerol, fructose, glucose, starch, sorbitol and sucrose. Preferred co-substrates are naphthalene and glycerol. Particularly preferred is naphthalene. If naphthalene is used, the preferred concentration range is 0.2 to 0.4 mg/l.

In a preferred embodiment of the present invention, the fermentation broth comprises at least one nitrogen sourceselected from the group consisting of inorganic ammonia salts, yeast powder and peptone. Particularly preferred it is an inorganic ammonia salt. A preferred inorganic ammonia salt is NH₄Cl. The concentration of the inorganic ammonia salt is preferably 0.5 g/l to 2 g/l.

A preferred fermentation broth comprises:
0.2 g/L 1-naphthol;
0.2 g/L naphthalene;
1 g/L NH₄Cl;
10 g/L NaH₂PO₄•2H₂O;
3 g/L K₂HPO₄•12H₂O;
0.5 g/LNaCl;
0.5 g/L FeSO₄•7H₂O;
0.5 g/L MgSO₄•7H₂O.

The pH during incubation is maintained between 6.0 and 8.0. The preferred pH is between 6.5 and 8.0. Especially preferred is the range from pH 7.0 to pH 8.0.

As the strain isolated in the study underlying the present invention does not require induction in order to synthetize 1,5-dihydroxynaphthalene, it is preferred to perform the method of the present invention without induction. The term "induction" refers to the addition of compounds which do not serve as substrate or nutrient to the bacteria and have the sole purpose of inducing the expression of enzymes convert naphthalene or 1-naphthol into 1,5-dihydroxynaphthalene. More preferably, the term "without induction" means that no acetylsalicylic acid is added to the fermentation broth before or during method step b).

In another embodiment, the present invention relates to the use of *Pseudomonas guariconensis njensis* for the manufacture of 1,5-dihydroxynaphthalene. It is preferred to use *P. njensis* 2-40.

In yet another embodiment the present invention relates to *Pseudomonas guariconensis njensis.* Particularly preferred is *P. njensis* 2-40 which has been derived from *Pseudomonas guariconensis njensis* by mutagenesis.

All definitions given above for the method of the present invention also apply to this embodiment

The following examples are merely intended to illustrate the present invention. They shall not limit the scope of the claims in any way.

### Examples

### Medium

The medium comprised:
0.2 g/L 1-naphthol;
0.2 g/L naphthalene;
1 g/L NH₄Cl;
10 g/L NaH₂PO₄•2H₂O;
3 g/L K₂HPO₄•12H₂O;
0.5 g/LNaCl;
0.5 g/L FeSO₄•7H₂O;
0.5 g/L MgSO₄•7H₂O

Fermentation in the above-described medium for 30 hours at 30 °C resulted in a titer of 1,5-dihydroxynapthalene of 0.37mg/L for the wild type and 1.47mg/L for *P. njensis* 2-40.

**Table 1: Effects of co-substrate culture on the growth of strain and the sythesis of 1,5-dihydroxynaphthalene**

| Carbon source | OD₆₀₀ | 1,5-DHN [mg/L] |
|---|---|---|
| Glucose | 4.53±0.01 | 1.09±0.17 |
| Glycerol | 4.72±0.31 | 0.89±0.15 |
| Saccharose | 3.91±0.13 | 1.40±0.10 |
| Fructose | 4.49±0.07 | 1.18±0.05 |
| Starch | 4.13±0.12 | 1.31±0.12 |
| Sorbitol | 4.07±0.22 | 1.41±0.04 |
| Naphthalene | 4.12±0.01 | 1.57±0.08 |
| Control | 4.03±0.02 | 1.42±0.12 |

**Table 2: Effects of the naphthalene concentration on the strain growth and the sythesis of 1,5-dihydroxynaphthalene**

| Concentration [g/L] | OD₆₀₀ | 1,5-DHN [mg/L] |
|---|---|---|
| 0.1 | 4.41±0.13 | 0.84±0.02 |
| 0.2 | 4.22±0.08 | 1.53±0.09 |
| 0.4 | 4.01±0.24 | 1.68±0.08 |
| 0.6 | 3.75±0.04 | 1.12±0.06 |
| 0.8 | 3.14±0.09 | 0.47±0.05 |
| 1 | 3.05±0.05 | 0.40±0.02 |

**Table 3: Effects of various nitrogen sources on the strain growth and the sythesis of 1,5-dihydroxynaphthalene**

| Nitrogen source | OD₆₀₀ | 1,5-DHN [mg/L] |
|---|---|---|
| Tryptone | 2.61±0.13 | 0.77±0.06 |
| Yeast powder | 1.37±0.03 | 0.89±0.07 |
| Beef extract | 1.50±0.15 | 0.29±0.02 |
| Yeast extract | 3.22±0.18 | 0.60±0.03 |
| Corn steep liquor | 5.83±0.14 | 0.35±0.07 |
| Control | 4.07±0.09 | 1.59±0.04 |

## Claims

1. Use of *Pseudomonas guariconensis njensis* for the manufacture of 1,5-dihydroxynaphthalene.

2. The use according to claim 1, wherein a strain of *Pseudomonas guariconensis njensis* as defined by CCTCC M 2021615 is used.

3. The use according to claim 1, wherein a strain of *Pseudomonas guariconensis njensis* as defined by CCTCC M 2021614 is used.

4. Use according to any one of claim 1 to 3 without induction.

5. Method for producing 1,5-dihydronaphthalene comprising the steps of
a) Adding microbial cells belonging to *Pseudomonas guariconensis njensis* to a fermentation broth comprising 1-Naphthol or Naphthalene or a mixture of 1-Naphthol and Napthalene; and
b) incubating the cells at a temperature between 30 and 40 °C for at least 3 hours.

6. The method according to claim 5, wherein a strain of *Pseudomonas njensis* as defined by CCTCC M 2021615 or CCTCC M 2021614 is used.

7. The method according to claim 5 or 6, wherein 1-naphthol is used and a co-substrate selected from the group consisting of naphthalene, glycerol, fructose, glucose, starch, sorbitol and sucrose is added to the fermentation broth before or during method step b).

8. The method of claim 7, wherein the co-substrate is glycerol.

9. The method of any one of claims 5 to 8, wherein in the fermentation broth comprises at least one nitrogen source selected from the group consisting of an inorganic ammonia salt, yeast powder and peptone.

10. The method of claim 9, wherein the nitrogen source is an inorganic ammonia salt.

11. The method of claim 10, wherein the inorganic ammonia salt is NH₄Cl.

12. Microorganism as defined by CCTCC M 2021615 or CCTCC M 2021614.
